# EUROPÄISCHE PATENTANMELDUNG

(11) **EP 1 201 181 A1**
(43) Veröffentlichungstag der Anmeldung: **02.05.2002**
(21) Anmeldenummer: 00810984.5
(22) Anmeldetag: 25.10.2000
(51) Int. Cl.: A61B 3/024

(54) **Verfahren zum Prüfen visueller Funktionen eines menschlichen Auges sowie Perimeter zur Durchführung des Verfahrens**

(71) Anmelder: Interzeag AG, CH-8952 Schlieren (CH)
(72) Erfinder: Jäggi, Peter, 5452 Oberrohrdorf (CH)
(74) Vertreter: Groner, Manfred

(57) **Zusammenfassung**

Zum Prüfen visueller Funktionen des menschlichen Auges (6) ist dieses an einer bestimmten Beobachterstelle (10) in Richtung einer Beobachtungsachse (9) zu fixieren. Mit einer Lichtquelle (13) zugeordnete Mittel dienen zur zeitlich gestaffelten Erzeugung von Stimuli an wählbaren Orten im Umfeld einer auf der Beobachtungsachse (9) angeordneten Fixationsmarke (32, F). Mit einer Kamera (34) wird die Augenposition erfasst. Eine Abweichung einer Augenposition von der definierten Fixationsmarke wird selbsttätig ermittelt. Aufgrund der ermittelten Abweichung im Perimeter (1) wird eine automatische Korrektur durchgeführt.

Vorzugsweise wird aufgrund der ermittelten Abweichungen die Positionen der Testorte korrigiert.

## Beschreibung

Die Erfindung betrifft ein Verfahren zum Prüfen visueller Funktionen eines menschlichen Auges mittels eines Perimeters, das an einer bestimmten Beobachterstelle in Richtung einer Beobachtungsachse orientiert ist, mit einer Lichtquelle zugeordneten Mitteln zur zeitlich gestaffelten Erzeugung von Stimuli an wählbaren Orten im Umfeld einer auf der Beobachtungsachse angeordneten Fixationsmarke, mit einem Rechner und mit einer Kamera zur Beobachtung des Auges.

Durch die CH-A-677 599 des Anmelders ist ein Perimeter bekannt geworden, bei dem die Augenposition des Probanden mittels einer Kamera erfasst wird. Wird eine zu grosse Abweichung von der idealen Blickrichtung festgestellt, wird der Untersuchungsablauf unterbrochen, der Proband auf die Abweichung aufmerksam gemacht und neu plaziert. Während einer Untersuchung können mehrere solche Unterbrechungen vorkommen. Durch diese Unterbrechungen verzögert sich der Untersuchungsablauf erheblich und die Untersuchungsdauer wird entsprechend verlängert.

Der Erfindung liegt die Aufgabe zugrunde, ein Verfahren zu schaffen, bei dem die genannten Nachteile vermieden sind und bei dem somit die Untersuchungsdauer nicht durch Unterbrechungen verlängert wird.

Die Aufgabe ist beim gattungsgemässen Verfahren dadurch gelöst, dass mit der Kamera die Augenposition erfasst wird, dass die Abweichung der Augenposition von der definierten Fixationsmarke selbsttätig ermittelt wird und dass aufgrund der ermittelten Abweichung eine automatische Korrektur durchgeführt wird. Beim erfindungsgemässen Verfahren werden die Koordinaten der Testorte aufgrund der ermittelten Abweichungen korrigiert, so dass sich eine Neupositionierung des Probanden erübrigt. Vor jeder Darbietung eines Stimulus wird mittels der Kamera die Blickrichtung bzw. die Position der Pupille des untersuchten Auges ermittelt. Wird eine Abweichung festgestellt, so wird für den darzubietenden Stimulus der entsprechende korrigierte Testort berechnet. Damit wird unabhängig von der Blickrichtung des Probanden der Stimulus relativ zum Gesichtsfeld immer an derselben Stelle dargeboten. Wie erwähnt, erübrigt sich damit eine Unterbrechung der Untersuchung zur Neupositionierung des Probanden. Denkbar ist auch eine Ausführung, bei welcher eine Korrektur erst nach der Darbietung der Stimuli durchgeführt wird. In beiden Fällen wird der Proband währen der gesamten Untersuchung nicht durch eine Unterbrechung gestört.

Ein Ausführungsbeispiel der Erfindung wird nachfolgend anhand einer Zeichnung näher erläutert. Es zeigen:
- Fig. 1: schematisch ein Perimeter zur Durchführung des Verfahrens,
- Fig. 2: schematisch ein Gesichtsfeld mit der Darstellung eines Skotoms,
- Fig. 3: schematisch eine von der idealen Position abweichende Augenposition und
- Fig. 4: schematisch die Korrektur eines Stimulus-Testortes.

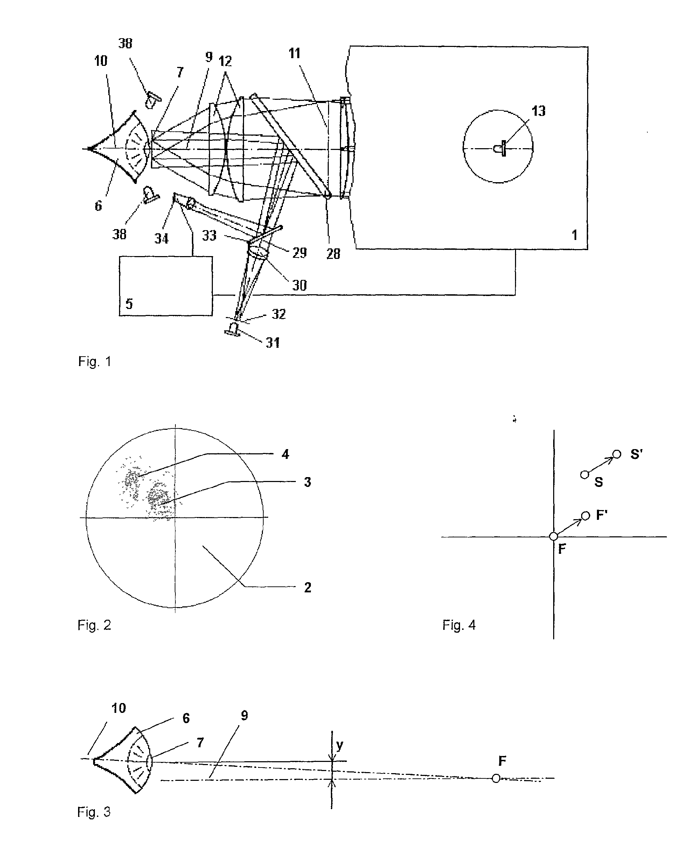

Die Fig. 1 zeigt ein Perimeter 1, in dem an einer bestimmten Beobachterstelle das zu untersuchende Auge 6 in einer Beobachtungsrichtung fixiert wird. Dem Auge werden während einer Untersuchung mit geeigneten Mitteln in zeitlicher Staffelung Stimuli im Umfeld einer Beobachterachse 9 präsentiert. Das Perimeter 1 wird mittels eines Rechners 5 gesteuert. Zur Offenbarung wird hier auch auf die CH-A-677 599 A5 des Anmelders hingewiesen.

Um dem Beobachterauge 6 die Orientierung mit Bezug auf die Beobachterachse 9 zu erleichtern, wird dem Probanden auf diese Achse 9 eine hell leuchtende Marke (Fixationsmarke) als Orientierungshilfe angeboten. Dazu dient ein zwischen einer Zwischenebene 11 und dem Okular 12 angeordneter, teilweise lichtdurchlässiger, gegen die Beobachterstelle 10 gerichteter Umlenkspiegel 28. Den Schnittpunkt zwischen dem Umlenkspiegel 28 und der Beobachterachse 9 schneidet weiter die optische Achse 29 von zwei Linsen 30, die achsial mit einer Lichtquelle 31 und einer Blende 32 ausgerüstet ist. Die Blende 32 bestimmt Form und Grösse der Fixationsmarke.

Über einen weiteren in der optischen Achse 29 angeordneten Umlenkspiegel 33, der für sichtbares Licht durchlässig und für infrarotes Licht reflektierend ist, ist eine IR-empfindliche CCD-Kamera auf die Beobachterachse 9 geschaltet, mit der das Auge 6 während einer Untersuchung betrachtet werden kann. Zu diesem Zweck wird das Auge 6 mit IR-LEDs 38 für den Probanden unsichtbar beleuchtet. Das von der Kamera 34 erfasste Bild wird im Rechner 5 ausgewertet. Hierbei wird die Position der Pupille 7 des Auges 6 ermittelt und die Abweichungen von der korrekten Blickrichtung bzw. Position werden berechnet. Eine solche mögliche Abweichung in der Blickrichtung zeigt schematisch die Fig. 3. Die Pupille 7 des Auges 6 befindet sich hier oberhalb der optischen Achse 9. Auch wenn das Auge 6 hier die Fixationsmarke 32 korrekt fixiert, würde beispielsweise ein Skotom 3 gemäss Fig. 2 an einer falschen Stelle, beispielsweise bei 4 gefunden.

Zur Korrektur der genannten Abweichung wird nun die Position der Stimulus-Testorte um die berechneten Werte, beispielsweise um den Wert y gemäss Fig. 3, korrigiert. Die Korrektur wird nötigenfalls für jeden Stimulus und in x- und y-Richtung durchgeführt. Ändert sich während einer Untersuchung die Blickrichtung mehrmals, so wird entsprechend mehrmals die Position des darzubietenden Stimulus korrigiert. Die Fig. 4 zeigt schematisch eine solche Korrektur eines Stimulus S. Die Fixationsmarke F steht im Nullpunkt des Koordinatensystems. Ist das Auge 6 korrekt positioniert und ist die Blickrichtung genau auf der optischen Achse 9, stellt die Kamera 34 keine Abweichung fest und entsprechend wird ein bezüglich seiner Position nicht korrigierter Stimulus S dargeboten. Bei einer Abweichung der Blickrichtung, beispielsweise fixieren auf den Punkt F' gemäss Fig. 4, wird diese Abweichung von der Kamera 34 erfasst und im Rechner 5 zu einem Korrekturwert umgerechnet. Aufgrund dieses Wertes wird ein Stimulus S' an einem entsprechend korrigierten Ort dargeboten. Der Stimulus S' wird somit trotz der abweichenden Blickrichtung relativ zum Gesichtsfeld des Probanden an der korrekten Stelle dargeboten.

## Patentansprüche

1. Verfahren zum Prüfen visueller Funktionen des menschlichen Auges (6) mittels eines Perimeters (1), das an einer bestimmten Beobachterstelle (10) in Richtung einer Beobachtungsachse (9) zu fixieren ist, mit einer Lichtquelle (13) zugeordneten Mitteln zur zeitlichen gestaffelten Erzeugung von Stimuli an wählbaren Orten im Umfeld einer auf der Beobachtungsachse (9) angeordneten Fixationsmarke (32, F), mit einem Rechner (5) und mit einer Kamera (34) zur Beobachtung des Auges (6), **dadurch gekennzeichnet, dass** mit der Kamera (34) die Augenposition erfasst wird, dass die Abweichung einer Augenposition von der definierten Fixationsmarke selbsttätig ermittelt wird und dass aufgrund der ermittelten Abweichung im Perimeter (1) eine automatische Korrektur durchgeführt wird.

2. Verfahren nach Anspruch 1, **dadurch gekennzeichnet, dass** aufgrund der ermittelten Abweichungen die Positionen der Testorte korrigiert werden.

3. Verfahren nach Anspruch 2, **dadurch gekennzeichnet, dass** für jede Darbietung eines Stimulus nötigenfalls eine entsprechende Korrektur der Position des Testortes durchgeführt wird.

4. Perimeter zur Durchführung des Verfahrens gemäss Anspruch 1, **dadurch gekennzeichnet, dass** es eine Kamera (34) zur Beobachtung des Auges (6) eines Probanden aufweist, dass die Kamera (34) mit einem Rechner (5) verbunden ist und dieser Rechner Mittel aufweist, um eine Abweichung von einer definierten Fixationsmarke in einen Korrekturwert umzuwandeln.
